## Europäisches Patentamt
## European Patent Office
## Office européen des brevets

(19)

(11) Publication number: **0 102 121**
**A1**

(12)

# EUROPEAN PATENT APPLICATION

(21) Application number: **83201201.7**

(22) Date of filing: **18.08.83**

(51) Int. Cl.³: **G 21 G 1/04**

(30) Priority: **26.08.82 NL 8203349**

(43) Date of publication of application:
**07.03.84 Bulletin 84/10**

(84) Designated Contracting States:
**AT BE CH DE FR GB IT LI NL SE**

(71) Applicant: **Byk-Mallinckrodt CIL B.V.**
**Westerduinweg 3**
**NL-1755 LE Petten(NL)**

(72) Inventor: **De Jong, Rudolf Barend Jan, Drs.**
**c/o OCTROOIBUREAU ZOAN B.V. Apollolaan 151**
**NL-1077 AR Amsterdam(NL)**

(74) Representative: **Swaters, Pieter D., Drs. et al,**
**Octrooibureau ZOAN B.V. Apollolaan 151**
**NL-1077 AR Amsterdam(NL)**

(54) Shielding device for a reservoir comprising a radioactive material.

(57) The invention relates to a shielding device for a reservoir comprising a radioactive material and having an inlet and an outlet aperture, in particular a column for a radio-isotope generator, comprising a lead cover for the reservoir in which a closable access for the reservoir is recessed. The shielding device furthermore is provided with means which the device can be moved forward.

EP 0 102 121 A1

Shielding device for a reservoir comprising a radioactive material.

The invention relates to a shielding device for a reservoir comprising a radioactive material and having an inlet and an outlet aperture, in particular a column for a radio-isotope generator, comprising a lead cover for the reservoir in which a closeable access for the reservoir is recessed.

A radioisotope generator is to be understood to mean herein a device for generating a radioactive isotopes comprising liquid. Such a liquid is prepared by eluting a column in which a parent isotope is present which produces a daughter isotope by decay. In this elution only the daughter isotope is eluted from the column by means of a suitable eluent.

Radioactive isotopes having a half-life up to a few days are frequently used in medicine for diagnostic purposes. One radioactive isotope frequently used for diagnostic examinations is technetium-99m. However, for certain applications, for example, for cardiological examinations, the comparatively long half-life of technetium--99m, namely 6 hours, is a disadvantage. As a result of this the radioactive material remains circulating in the body for a long period of time, so that an immediate repetition of a certain diagnostic examination with the same isotope is not possible.

However, very short-living radioactive isotopes having a half-life up to a few minutes, for example gold-195m, rubidium-82 and krypton-81m, are suitable for such above--mentioned examinations. Krypton-81m is used for lung function examinations, while rubidium-82 and gold-195m have proved suitable for blood circulation studies. An interesting application of gold-195m was described recently in Netherlands non-prepublished Patent Application 8201591 in the name of Applicants.

Gold-195m is an isotope having a half-life of 30.6 sec. and emits gamma rays of 261 keV which, due to the energy and intensity, are suitable to enable a good observation with apparatus usual for this purpose, for example, a gamma camera.

It is known from Netherlands Patent Application 8002235 in the name of Applicants to generate gold-195$\underline{m}$ from the radioactive parent isotope mercury-195$\underline{m}$ in a satisfactory manner. This process is preferably carried out in a so-called radio-isotope generator, in this case a Hg/Au generator, from which the use can withdraw a quantity of radioactive isotope-containing liquid at any desired instant. Such an instantaneous production is of great practical importance due to the rapid decay of the comparatively short-living isotope.

An improved method of preparing gold-195$\underline{m}$ is described in Netherlands non-prepublished Patent Application 8202407 also in the name of Applicants.

In view of the high radiation intensity, extensive safety measures have to be taken to shield the parent isotope present in the generator. Therefore the generator comprises a lead screening jacket which provides a sufficient safety upon storage and transport. The screening jacket surrounding the generator is generally considered to be an insufficient safety against radioactive radiation for hospital or laboratory personnel who are regularly in the direct proximity of the generator. It is therefore necessary to surround the generator with an extra lead shielding device.

Such a device should not only provide a good shielding from radioactive radiation, but, in connection with the necessity of a regular replacement, should also be readily accessible for the reservoir with radioactive material, in particular the generator column.

Therefore, various shielding devices are known from literature substantially all of which are destined for a column for generating technetium-99$\underline{m}$, a radioactive isotope having a comparatively long half-life, and all of which are fixedly arranged.

When a very short-living radioactive isotope is used for diagnostic purposes, the time between the preparation of the isotope and the administration to a patient should be minimized. In the case of the above-mentioned gold-195m isotope having a half-life of approximately 30 seconds, it is very much desired, if not necessary, to administer said isotope directly from the generator to the patient. In a clinic in which radioactive isotopes for diagnostic purposes are used, the apparatus necessary for detection, for example, a gamma camera with special collimator and a computer, is usually fixedly arranged. For a radiodiagnostic examination the patient is then brought (wheeled) to the detection apparatus.

It is therefore obvious to give the very short-living material to be used for the examination, in particular a generator for producing a very short-living isotope, a fixed place close to the detection apparatus. It is feasible that high requirements as regards the shielding from radioactive radiation have to be imposed upon such a device beside the patient to be examined ("bed-side arrangement"). In fact, not only the hospital personnel familiar with handling radioactive material will have to be present near the radiation source for a longer period of time, but also other personnel accompanying the patient will have to be shielded from unnecessary radioactive radiation. Moreover it is of utmost importance for the examination that the source of radiation should be shielded carefully from the gamma camera which is very disturbance--sensitive to background radiation.

A fixed arrangement as suggested above which would satisfy these requirements, however, has important practical disadvantages, namely:

(1) it is not possible to move the device around the patient's bed. This is a disadvantage because in examinations with very short-living radioactive isotopes, the organ, for example, the heart, has to be inspected usually in various directions by means of the gamma camera, so as to gain optimum insight in the function of the organ. A fixed bed-side arrangement of the radioactive material to be administered considerably

-4-

restricts the possibilities of moving the bed with the patient with respect to the gamma camera. This disadvantage is the larger since, for reasons which will be stated hereinafter, the connection between the source of the radioactive material and the patient should be as short as possible.

(2) Assembling of the device must take place for the greater part under aseptic conditions because the radioactive material must be introduced into the patient's body directly and cannot be previously subjected to a separate sterilization.
Because the device with radioactive material, in particular the radio isotope generator, will be used for a longer period of time, such an assembling should be carried out under so-called laminar flow conditions, and therefore requires provisions which are particularly difficult to realize in an examination room.

(3) Another important disadvantage relates to the working with radioactive material upon assembling the device. As a matter of fact, the shielding from radioactive radiation is not yet optimum during the assembly, so that such an assembly, in which large quantities of not yet optimally shielded activity are handled, should therefore take place in a so-called hot-lab of a nuclear medical department of a clinic and not in an examination room for patients where in addition disturbance-sensitive detection apparatus is arranged.

It is the object of the present invention to provide a shielding device for a reservoir comprising radioactive material, in particular a column for a radioisotope generator, which does not exhibit the above-mentioned disadvantages.

For that purpose, the shielding device according to the invention is provided with means with which the device can be moved forward. The complete device comprising radioactive material can now be assembled in suitable rooms intended for this purpose and can then be wheeled to

-5-

the examination room beside the patient's bed. Because the shielding device can be freely shifted, the device can be moved at will around the patient during the examination. Such a movable shielding device for a column for a radio-isotope generator is moreover more flexible because the device can be used, if desired, for any generator, for example, a rubidium-krypton-81$\underline{m}$, a strontium-rubidium 82 or a mercury-gold 195$\underline{m}$ generator.

It is of course necessary that the shielding device should also satisfy all conventional safety requirements in addition to the above-mentioned radiological safety requirements. This involves, for example, that the device should be sufficiently stable and be protected as well as possible from calamities, for example, a fire; in the latter case, of course, it should be prevented that the radioactive radiation can pass the shielding device and enter the examination room.

Preferably the device in addition comprises provisions for the safe handling of radioactive material, such as a receptacle for waste fluid, a work-top, etc. These provisions enable the user to carry out various manipulations with radioactive materials at different places without risky manually displacing these materials, because, as a matter of fact, the device can be moved forward.

On its lower side the shielding device preferably comprises at least three, preferably five, casters to be able to easily turn and manoeuvre the heavy device in the restricted space around a patient. As a result of the lead cover, a shielding device according to the invention approaches a weight of approximately 360 kg.

Furthermore it is desired to provide the device with a grip at a height which is suitable for hand-movement. For this purpose, a grip consisting of a circumferential tubular or rod-shaped member connected to the outside of the lead cover has proved particularly suitable. When such a grip having no projections is used, it is avoided that components of the device or connections can be drawn along or loose during movement of the shielding device.

When using the device it is often necessary to temporarily store radioactive waste material. For example, when a gold-195m generator is used, the generator column must first be rinsed several times with eluent before an eluate is obtained having a composition which is sufficiently constant for administration to a patient. It is therefore advantageous that the device moreover comprises a separate lead-shielded space for a receptacle for radio--active waste material.

Because the radioactive liquid has to be introduced directly into the patient's body, the means for doing this are preferably connected on or to the shielding-device.

In a suitable embodiment the shielding device according to the invention comprises a base in which the means to move the device are present, a central part of reduced outside diameter in which the lead cover for the reservoir containing radioactive material is present, and a top part which comprises: the lead closure for the access in the cover, the grip, the access to the shielded space for the waste reservoir and the means to introduce a radioactive liquid into a patient's body.

As a result of the large diameter of grip and base as compared with that of the reservoir shielded by means of a lead cover, the distance between the radiation source and the operating personnel is increased, for example, by a factor of approximately 2. As a result of this the radiation received is still further reduced, for example, by a factor of approximately 4 as compared with the radiation at the outer surface of the shielded reservoir.

Lead is vulnerable because is is a soft metal. Moreover, it has a low melting-point, $327^{\circ}C$, so that in the case of a fire, it will melt and drip away, thus allowing radioactive radiation to be released from the cover. Therefore, the lead cover for the reservoir consists preferably and in agreement with the requirements which are imposed upon the storage of radioactive material in various countries, of a lead vessel which is open at its top and which is enclosed between sheet material of iron or steel, protected on the outside against corrosion, or of stainless steel, while the open top end accessible for the reservoir can be closed by a lead lid provided with the same sheet material on the outside, an aperture for a connection between the reservoir and the means for introducing a radioactive liquid into a patient's body being present in the lid or between the vessel and the lid. The sheet material which can withstand high temperatures ensures sufficient safety for the ambience in the case of a calamity, for example, a fire, so that the lead shielding remains contained and no undesired radioactive radiation can get out of the shielding system.

It cannot always be avoided that a little radioactive liquid is spilled when installing or using the source of the radioactive material. Then it is difficult to thoroughly clean the vessel which forms part of the heavy shielding device. Therefore, a stainless steel vessel is preferably present between the substantially lead vessel and the reservoir, which stainless steel vessel comprises on its open top a radially outwardly projecting flange to which the lid can be sealingly connected.

The shielding device in accordance with the invention serves in particular for shielding a radio isotope generator. The provisions necessary upon eluting a generator column are preferably connected on or to the above-mentioned top part of the device, namely a reservoir for the eluent for the generator column which communicates with the column; means for pumping or injecting the eluent out of the eluent reservoir into the column; means for bringing the resulting eluate out of the column into a patient's body; means for adding a rinsing of formulating

-8-

liquid to the eluate; and a tube which is connected on one side to the means for adding the rinsing or formulating liquid and which on its other side has a member which can be connected to an auxiliary means to admit liquids to the blood vessels or body cavities of a patient.

In order to be able to handle all operating members easily, rapidly and safely, a connection and operating block or tray is connected to the top part, in which block are accommodated injection means for the eluent and the eluate, valves to prevent undesired directions of flow of liquids, cocks to enable or block the passage of liquids, and connection provisions for the means provided in the block both mutually and to the tubes which are connected to the reservoirs, the column and the auxiliary means to be used for the administration to a patient. Preferably the operating block or tray is attached on top of the lid of the lead vessel and the lid is provided with a bore to let pass connecting tubes from the generator to the auxiliary means for injection and from the auxiliary means to the waste fluid receptacle thereby shielding the environment as far as possible from radiation emanating from these tubes when radioactive liquid passes through them. The above embodiment has the advantage that an optimum safety can be reached inspite of the excess pressure at which generally the radioactive liquid is administered to a patient. Moreover, the path which the eluate has to cover, hence the distance between the generator and the patient, can be kept as short as possible. This latter is of importance in particular because, when very short-living radio-isotopes are used, high requirements are generally imposed upon the volume to be administered to the patient and in which the radioactive material is present. As described in the above-mentioned Netherlands Patent Application 8201591, repeated administrations within a short period of time are necessary for various applications. In order to enable such examinations, the volume in which the reactivity is present must be as small as possible.

The invention will now be described in greater detail with reference to an embodiment which is shown in the accompanying drawings.

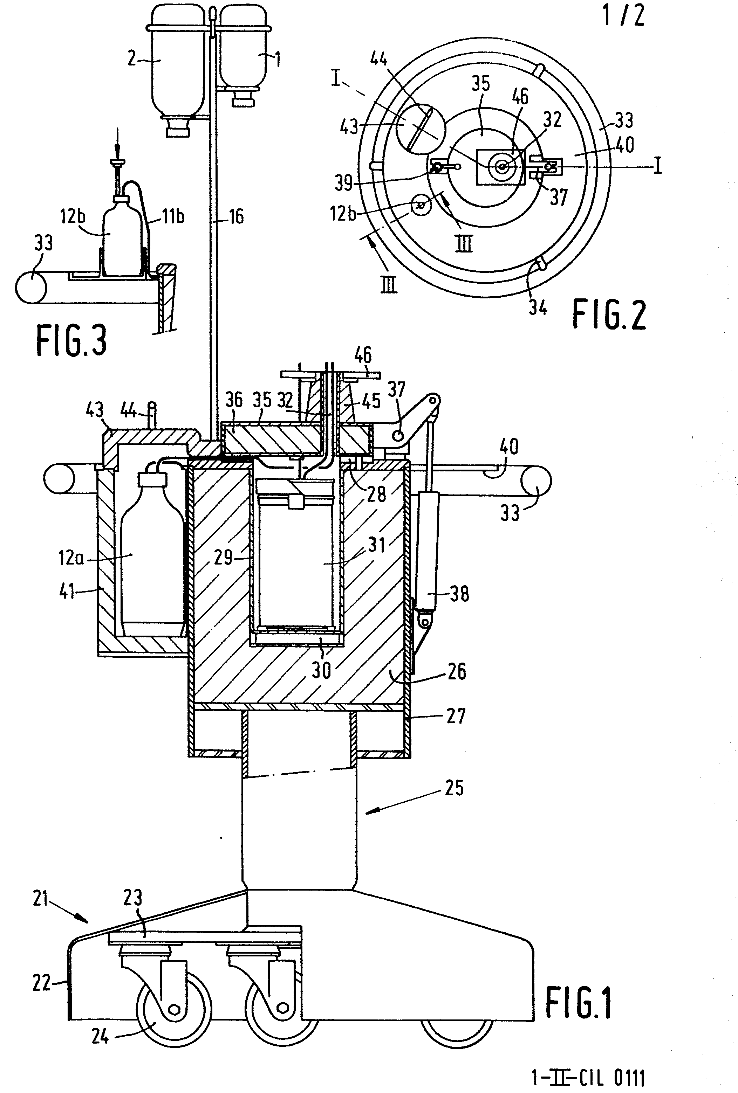

Figure 1 is a side-view of a shielding device according to the invention; figure 2 shows the same shielding device from top. Figure 1 is for the greater part a longitudinal sectional view of the shielding device taken on the line I-I of fig. 2, viewed in the direction of the arrows. Figure 3 is a longitudinal sectional view of a part of the device taken on the line III-III of fig. 2.

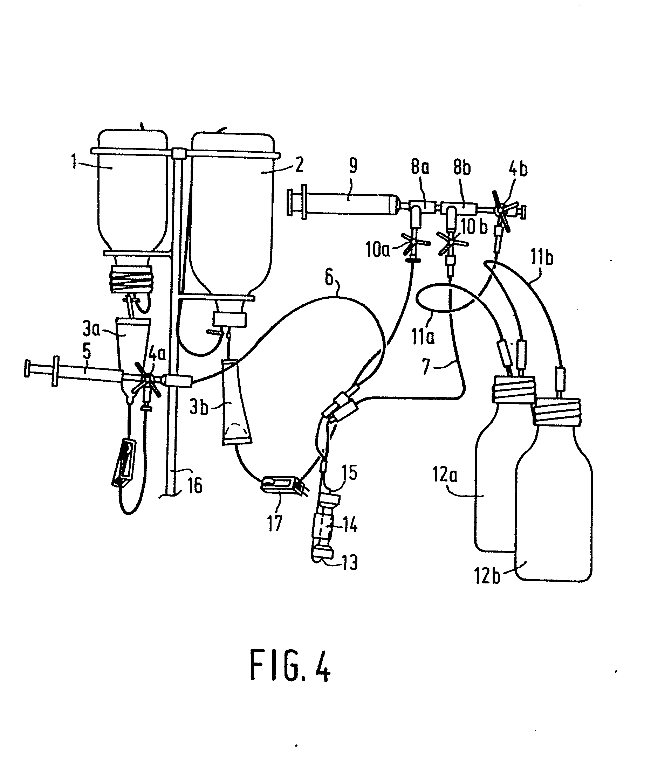

The operation of the device will be described in detail with reference to figure 4. Figure 4 shows an exploded view of a part of the device.

The base 21 of the screening device shown in Figure 1 comprises a base plate 23 which is hooded with a stove-enamelled sheet iron cap 22 below which five casters 24 are connected so as to be rotatable.

The central part 25 is mounted on said base plate and comprises a lead vessel 26 which is enclosed between stove-enamelled sheet iron 27. A second vessel 29 which is manufactured from stainless steel and comprises a radially outwardly projecting flange 28 is provided in the vessel. The generator 31 is placed in vessel 29. Between the bottoms of the vessels 27 and 29 a space 30 remains in which heating elements, for example a heating plate, can be accommodated. As described in the above-mentioned Netherlands Patent Application 8202407 it may be useful when certain radio isotope generators are used, for example, a gold-195m generator, to heat the generator column during the elution. If desired, a bore may be recessed in vessel 26 for leading through a supply for the heating means.

As shown in figures 1 and 2 a grip 33 in the form of a circumferential tube which is connected to the vessel by means of three spoke-shaped elements 34 is provided around the top part of the device. The vessel 26 can be closed on its top side by means of a lead lid 36 mounted in stove-enamelled sheet iron 35 and connected to the vessel so as to be pivotable at 37. For compensation of the weight of the lid, a spring mechanism 38 is provided. The lid can be clamped sealingly on the vessel (flange 28) by means of a clamping lock 39 provided with a handle. A bore 32 is present in lid 36 for leading through two connection tubes, the outlets of which are framed in a suitable mount 45, comprising a steel tube encased in lead, erected on the lid of the lead vessel and forming a base for an operating block or tray. Between the circumferential grip and the upper edge of the lead vessel, a circumferential stainless steel top 40 having upright edges is present on which auxiliary means necessary for using the device can be placed.

A small lead vessel 41, also mounted in stove-enamelled sheet iron, for a receptacle 12a for waste material is present in an aperture of the top 40, which vessel is connected to the large vessel 27 and can be closed by means of a lead lid 43 provided with a grip 44.

On-top of mount 45 is connected an operating block or tray 46 in or on which two syringes can be accomodated, as well as other auxiliary means needed during operation of the device.

Figure 3 shows a waste overflow bottle 12b placed on top 40. The inlet of the overflow bottle is connected to the outlet tube 11b of receptacle 12a.

As shown in Figure 1, two reservoirs 1 and 2 for eluent and rinsing or formulating liquid, respectively, are clamped in a stand 16 mounted on the edge of vessel 27.

As shown in Figure 4, two syringes 5 and 9 provided on their front sides with connection means in the form of Luer cones are connected to three-way cocks, the former directly to a three-way cock 4a and the latter to a three-way cock 4b via two valves 8a and 8b.

The use of the device shown will be explained with reference to figure 4. All connections between the various components, for the greater part tube connections

-11-

and Luer connections, are produced under laminar flow conditions.

During operation of the device the tube connections are provided between eluent reservoir 1 and an outlet of three-way cock 4a, the inlet aperture of the generator column 13 and the other outlet of three-way cock 4a, the reservoir with rinsing or formulating liquid 2 and valve 8a, the drain aperture of the generator column 15 and valve 8b, the receptacle for waste fluid 12a and an outlet of three-way cock 4b and the auxiliary means to be used for administration to a patient and the other outlet of three-way cock 4b.

When the device is used, first three-way cock 4b is opened to communicate the eluate duct 7 through cock 10b and valve 8b with the waste fluid receptacle 12a. Overflow bottle 12b is connected to receptacle 12a through a tube 11b and serves as an extra safety. By means of three-way cock 4a, syringe 5 is communicated with eluent reservoir 1, after which the syringe is filled with 2 ml of eluent. Eluent reservoir 1 and rinsing agent reservoir 2, clamped in stand 16, are provided with dropping chambers 3a and 3b. After opening the cock 10a, syringe 9 is filled with a saline solution from reservoir 2 (through valve 8a); the tube is then closed by clamping by means of clamb 17. After having turned three-way cock 4a, the contents of syringe 5 are injected through tube 6 into the generator column 14 at 13; after-rinsing is carried out with 2 ml of saline solution from syringe 9. All the wash liquid (eluate) rinsed through the column and leaving the generator column at 15, as well as the rinsing liquid is collected through tubes 7 and 11a in the waste receptacle 12a.

After having repeated this operation several times, the generator is ready for connection to a patient. For that purpose, a sterile tube, connected to three-way cock 4b, is filled with a saline solution from syringe 9 after opening said valve, and is then connected to an

-12-

auxiliary means to administer the radioactive liquid to a patient, for example, a needle or a catheter. After having placed the patient in a suitable position below a gamma camera, the generator is eluted with 2 ml of eluent by means of syringe 5, the eluate being injected directly into the patient. All remaining radioactivity is then removed from the device by rinsing with 10 ml of saline solution from reservoir 2 by means of syringe 9.

The examination may be repeated any desirable number of times.

-13-

CLAIMS:

1. A shielding device for a reservoir comprising a radioactive material and having an inlet and an outlet aperture, in particular a column for a radio-isotope generator, comprising a lead cover for the reservoir in which a closable access for the reservoir is recessed, characterized in that the shielding device is provided with means with which the device can be moved forward.

2. A device as claimed in Claim 1, characterized in that the device comprises in addition provisions for the safe handling of radioactive material.

3. A device as claimed in Claim 1 or 2, characterized in that the device comprises on its lower side at least three, preferably five, casters.

4. A device as claimed in any of the preceding Claims, characterized in that the device comprises a grip, preferably consisting of a circumferential tubular or rod--shaped member connected to the outside of the lead cover.

5. A device as claimed in any of the preceding Claims, characterized in that the device comprises in addition a separate lead-shielded space for a reservoir for radioactive waste material.

6. A device as claimed in any of the preceding Claims, characterized in that the device is provided with means for introducing a radioactive liquid into a patient's body, while the environment is shielded as far as possible from radiation emanating from these means when radioactive liquid passes through them.

7. A device as claimed in Claim 6, characterized in that the device comprises a base in which the means to move the device are present, a central part of reduced outside diameter in which the lead cover for the reservoir containing the radioactive material is present, and a top part which comprises: the lead closure for the access in the cover, the grip, the access to the shielded space for the waste reservoir and the means to introduce a radioactive liquid into a patient's body.

8. A device as claimed in Claim 7, characterized in that the lead cover for the reservoir consists of a lead vessel which is open at its top and which is enclosed between sheet material of iron or steel treated externally against corrosion, or of stainless steel, while the open top end accessible for the reservoir can be closed by means of a lead lid provided on its outside with the same sheet material, an aperture for a connection between the reservoir and the means for introducing a radioactive liquid into a patient's body being present in the lid or between the vessel and the lid.

9. A device as claimed in Claim 8, characterized in that a vessel of stainless steel which at its open top side comprises a radially outwardly projecting flange to which the lid can be sealingly connected, is present between the substantially lead vessel and the reservoir.

10. A shielding device as claimed in any of the Claims 7-9 for a radio-isotope generator, characterized in that there are additionally connected on or to the top part: a reservoir for an eluent for the generator column which communicates with the column; means for pumping or injecting the eluent out of the eluent reservoir into the column; means to bring the resulting eluate out of the column into a patient's body; means to add a rinsing or formulating liquid to the eluate; and a tube which is connected on one side to the means for adding the rinsing or formulating liquid and which comprises on the other side a member which can be connected to an auxiliary means to admit liquid to blood vessels or body cavities of a patient.

11. A device as claimed in Claim 10, characterized in that a connection and operating block or tray is connected to the top part, in which block are accomodated injection means for the eluent and the eluate, valves to prevent undesired directions of flow of liquids, cocks to enable or block the passage of liquids, and connection means for the means accomodated in the block both mutually

-15-

and to the tubes which are connected to the reservoirs, the column and the auxiliary means to be used for administering to a patient.

FIG.3

FIG.2

FIG.1

FIG. 4

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | GB-A-1 234 020 (C.E.A.) <br> * Claim 1; figure 1; page 2, lines 83-90; page 3, lines 68-76 * | 1,2,7 | G 21 G 1/04 |
| A | GB-A-2 033 288 (BYK MALLINCKRODT) <br> * Claim 1; figure 1; page 3, lines 29-40 * | 1,2 | |
| A | US-A-3 710 118 (R.L. HOLGATE) <br> * Claims 1,2 * | 1,10 | |

TECHNICAL FIELDS SEARCHED (Int. Cl. ³)

G 21 G
G 21 F

The present search report has been drawn up for all claims

| Place of search <br> THE HAGUE | Date of completion of the search <br> 17-11-1983 | Examiner <br> NICOLAS H.J.F. |
|---|---|---|